# EUROPÄISCHE PATENTANMELDUNG

(11) **EP 4 406 488 A1**
(43) Veröffentlichungstag der Anmeldung: **31.07.2024**
(21) Anmeldenummer: 24154393.3
(22) Anmeldetag: 29.01.2024
(51) Int. Cl.: A61B 10/00, B01L 3/00

(54) **ADAPTER ZUM ÜBERFÜHREN UND AUFBEREITEN EINER MENSTRUATIONSAUSSCHEIDUNGEN ENTHALTENDEN PROBE IN EIN VERSCHLIESSBARES PROBENGEFÄSS**

(30) Priorität: 30.01.2023 DE 102023102184
(71) Anmelder: The smart period blood GmbH, 10119 Berlin (DE)
(72) Erfinder: Guenou, Isabelle, 10249 Berlin (DE); Santer, Miriam, 10997 Berlin (DE)
(74) Vertreter: Weisse, Renate

(57) **Zusammenfassung**

Ein Adapter (10) zum Überführen einer Blut enthaltenden Probe in ein verschließbares Probengefäß (34), ist dadurch gekennzeichnet, dass der Adapter (10) ein Adaptergehäuse (12) mit einer Aufnahmeöffnung (26) mit Abmessungen aufweist, die das manuelle Eingießen der Probe in die Aufnahmeöffnung (26) sicher erlauben; und wenigstens ein Filter (36, 38) in oder an dem Adaptergehäuse (12) vorgesehen ist, mit welchem Zellen, Gewebe, Sekret oder andere feste Bestandteile der Probe vom Blut abtrennbar ist.

## Beschreibung

### Technisches Gebiet

Die Erfindung betrifft einen Adapter zum Überführen einer Blut enthaltenden Probe in ein verschließbares Probengefäß. Die Erfindung betrifft ferner ein Verfahren zum Analysieren von Blut enthaltenden Proben mit den Schritten:
(a) Überführen des in der Probe enthaltenen Bluts in ein Probengefäß;
(b) Verpacken und Befördern des Probengefäßes zu einem Analysenlabor; und
(c) Durchführen der Analyse.

Mit Blutuntersuchungen kann das Vorliegen einer Vielzahl von Bestandteilen des Bluts quantitativ und qualitativ erfasst werden. Hierzu gehören Biomarker wie Element- und Molekülkonzentrationen, Enzyme, pH-Wert, Hormone, und vieles mehr.

Für die Blutuntersuchung wird dem Körper eine Blut enthaltende Probe entnommen. Die Entnahme venösen Blutes erfolgt typischerweise mit einer Kanüle in einer klinischen Umgebung durch qualifiziertes Personal. Dabei wird die Vene punktiert und das Blut über einen Schlauch in ein Röhrchen überführt. Das Röhrchen wird beschriftet oder mit einem aufklebbaren Barcode versehen und in ein Labor zur Analyse transportiert. Der Nadelstich kann zu Beschwerden wie Infektionen und lokalen Blutergüssen führen.

Die Entnahme von kapillarem Blut, etwa zur Blutzuckerselbstkontrolle, kann auch durch die zu untersuchende Person selbst durchgeführt werden. Dann ist die Blutmenge sehr gering. Sowohl die Entnahme von kapillarem, als auch von venösem Blut erfordert das Einstechen in die Haut und ist unangenehm.

Analysengeräte sind je nach Art der Analyse teuer. Sie sind daher nicht im Besitz der zu untersuchenden Person und häufig auch nicht in Arztpraxen vorhanden. Die Probe muss angemessen verpackt und zu einem Labor transportiert werden. Da Blut nicht stabil ist und insbesondere koaguliert und/oder mit dem Luftsauerstoff reagiert, muss bereits bei der Probennahme eine Probenaufbereitung erfolgen, mit der ein nicht-medizinisch geschulter Laie regelmäßig überfordert ist.

### Stand der Technik

Allgemein bekannt sind Blutentnahmeröhrchen, etwa aus https://www.bd.com/resource.aspx?IDX=25273 abgerufen am 21. November 2022. Dabei handelt es sich um Probengefäße in Röhrchenform, welche mit Chemikalien wie EDTA oder Citrat versehen sind. In Abhängigkeit der Zusätze sind die Verschlüsse nach der Norm EN14820 farbig kodiert. Man unterscheidet ein Aspirationssystem, bei dem mittels eines Kolbens, ähnlich wie bei einer Spritze, ein Unterdruck im Röhrchen erzeugt wird, von einem Unterdrucksystem, bei dem das Röhrchen Unterdruck aufweist und mit einer durchstechbaren Membran verschlossen ist. In beiden Fällen wird die Probe in das Röhrchen eingesaugt. Die bekannten Blutentnahmeröhrchen haben Durchmesser von 13 mm oder 16 mm und passen in handelsübliche Zentrifugen und andere Laborgerätschaften.

Es gibt Forschungsbeiträge, welche sich mit der Untersuchung von Menstruationsausscheidungen beschäftigen: Die Veröffentlichung von Fraser, I. S., McCarron, G., Markham, R. und Resta, T. "Blood and total fluid content of menstrual discharge". Obstet Gynecol 65, 194-198 (1985) offenbart die Zusammensetzung der Menstruationsausscheidungen. Die Veröffentlichung von Reed, B. G. und Carr, B. R. "The Normal Menstrual Cycle and the Control of Ovulation. in Endotext (eds. Feingold, K. R. et al.) (MDText.com, Inc., 2000) offenbart die Veränderung der Menstruationsausscheidungen mit dem Zyklus. Es konnte ferner gezeigt werden, dass Menstruationsausscheidungen sich als eine Quelle von Stammzellen eignet. Dies ist offenbart in den Veröffentlichungen von Meng, X. et al. "Endometrial regenerative cells: a novel stem cell population." J. Transl. Med. 5, 57 (2007); Chen, L., Qu, J., Cheng, T., Chen, X. und Xiang, C. "Menstrual blood-derived stem cells: toward therapeutic mechanisms, novel strategies, and future perspectives in the treatment of diseases. Stem Cell Res. Ther. 10, 406 (2019) und Mou, X. et al. "Menstrual blood-derived mesenchymal stem cells differentiate into functional hepatocyte-like cells. J Zhejiang Univ Sci B 14, 961-972 (2013).

In den zu obigen Veröffentlichungen gehörigen Forschungslaboren stehen eine Vielzahl von Instrumenten und Gerätschaften zur Verfügung, mit denen Menstruationsausscheidungen gesammelt, transferiert und analysiert werden können. Derartige Instrumente und Gerätschaften sind bei Laien normalerweise nicht vorhanden. Die Probennahme bei Blut enthaltenden Proben aus Menstruationsausscheidungen erfordert somit wie bei venösem Blut den Gang in ein Labor.

Für das Sammeln von Menstruationsausscheidungen sind Menstruationstassen bekannt. Wie bei der Verwendung von Tampons und Binden wird das mit Menstruationstassen gesammelte Blut in der Regel entsorgt. Ein Beispiel für eine Menstruationstasse ist in EP3 470 026 B1 offenbart. WO 2012/084987 A2 offenbart eine Binde zum Absorbieren u.a. von Menstruationsausscheidungen.

WO 2017/161378A1 offenbart eine Menstruationstasse zum Sammeln von Menstruationsausscheidungen für die Analyse. Die gesammelte Probe bleibt entweder in der Menstruationstasse, die mit einem Deckel verschlossen wird oder sie wird als Ganzes einschließlich Zellresten etc. in ein Probengefäß gegossen. Da die Menstruationstasse flexibel ist, besteht die Gefahr, dass sie beim Transport zusammengequetscht wird und Undichtigkeiten entstehen, durch welche die Probe austreten kann. Die Mehrfachverwendung der Menstruationstasse ist bei dieser Variante nicht möglich.

Das direkte Eingießen aus der Menstruationstasse in ein Probenröhrchen ist ebenfalls unvorteilhaft. Übliche Probenröhrchen haben einen vergleichsweise geringen Durchmesser von 13-16 mm. In der Probe befinden sich schleimige Zellen, Gewebe, Sekret, Partikel und Feststoffe, die das Gießen erschweren. Bei den geringen Durchmessern der Probenröhrchen besteht die Gefahr, dass Teile der Probe auf den Rand auftreffen oder vollständig verschüttet werden. Das ist unerwünscht. Eine verschüttete blutige Probe kann schwer entfernbare Flecken auf Kleidung und Umgebung hinterlassen.Die Bestandteile der Menstruationsausscheidungen, die nicht zum Blut gehören, können in der Zeit bis zur Analyse zu Veränderungen der Probe führen und die spätere Analyse stören.

Zusätzliche Hürden sind die Stabilität der Probe beim Versand und die Variation in den Bestandteilen, etwa Viskosität und Zellanteil. Beides ist bei Menstruationsausscheidungen problematischer als bei üblichen Blutproben und führt zu unzuverlässigen Laborergebnissen.

### Offenbarung der Erfindung

Es ist Aufgabe der Erfindung, die Probennahme und Teile der Probenvorbereitung zur Blutuntersuchung deutlich zu erleichtern und so auch für Laien zu erschließen. Erfindungsgemäß wird die Aufgabe gelöst durch einen Adapter zum Überführen einer Blut enthaltenden Probe in ein verschließbares Probengefäß, der dadurch gekennzeichnet ist, dass
(a) der Adapter ein Adaptergehäuse mit einer Aufnahmeöffnung mit Abmessungen aufweist, die das manuelle Eingießen der Probe in die Aufnahmeöffnung sicher erlauben; und
(b) wenigstens ein Filter in oder an dem Adaptergehäuse vorgesehen ist, mit welchem Zellen, Gewebe, Sekret oder andere feste Bestandteile der Probe vom Blut abtrennbar ist.

Die erfindungsgemäße Lösung erlaubt die Verwendung von Menstruationsblut für die Blutanalyse. Blut aus Menstruationsausscheidungen steht ohne Einstechen in die Haut in ausreichender Menge zur Verfügung. Mit dem Adapter können die gesammelten Menstruationsauscheidungen in ein Blutentnahmeröhrchen eingegossen werden. Solche Blutentnahmeröhrchen können handelsübliche Blutentnahmeröhrchen sein oder speziell entwickelte Blutentnahmeröhrchen mit den Abmessungen kommerziell verfügbarer Röhrchen. Der erfindungsgemäß größere Durchmesser der Aufnahmeöffnung, von beispielsweise wenigstens 2 cm, vorzugsweise wenigstens 2,5 cm und höchst vorzugsweise wenigstens 4 cm, erleichtert die Handhabung und mindert das Risiko, dass Probenteile verschüttet werden. Anders als bei bekannten Anordnungen zur Blutentnahme kann Menstruationsblut mit dem erfindungsgemäßen Adapter auch zuhause ohne zusätzliche Expertise in das Blutentnahmeröhrchen überführt werden. Durch den Filter werden weitere Anteile der Menstruationsausscheidungen, etwa Zellreste und dergleichen, von Beginn an abgetrennt. Dadurch wird das Risiko der Blockierung von Probenvorbereitungs- und Analysegeräten bei der Untersuchung des Blut enthaltenden Filtrats verringert und die Richtigkeit der Ergebnisse erhöht. Das Blutentnahmeröhrchen kann im Labor direkt verwendet und beispielsweise in eine Zentrifuge eingesetzt werden. Ein neuerliches Transferieren in andere Probengefäße ist nicht erforderlich, weil störende Feststoffe, Schleim etc. bereits herausgefiltert wurden.

Eine besonders vorteilhafte Anordnung sieht vor, dass der Adapter eine Nadel an der Unterseite des Adaptergehäuses umfasst, mit welcher eine an dem Probengefäß befindliche Membran durchstechbar ist, so dass das gefilterte Blut durch die Nadel in das Probengefäß überführbar ist.

Insbesondere kann vorgesehen sein, dass eine zumindest teilweise lösbare Schutzhülle und/oder eine Versiegelung an der Nadel vorgesehen ist. Dadurch werden Verunreinigungen an der Nadel und die Verletzungsgefahr vermieden. Die Menstruationsausscheidungen sind bereits gefiltert, wenn sie zur Nadel gelangen, so dass diese im Regelfall nicht verstopft wird. Die Membran am Probengefäß garantiert die Probennahme ohne Verunreinigungen. Besonders vorteilhaft ist es, wenn der Adapter mit einem Probengefäß zusammenwirkt, welches ein kommerziell verfügbares Probennahmeröhrchen ist. Beispiele für solche Probennahmeröhrchen werden u.a. unter den Handelsnamen Vacutainer, Vacuette, Kabevette oder Monovette vertrieben und sind allgemein bekannt. Sie können entweder als Aspirationssystem mit einem Kolben oder als Unterdrucksystem mit einer durchstechbaren Membran ausgebildet sein.

Der Adapter kann beispielsweise ganz oder teilweise konisch ausgebildet sein. Es kann aber auch vorgesehen sein, dass die Aufnahmeöffnung von einem Trichter gebildet ist, dessen Auslaufende in einem Behälter mündet, der einen geringeren Querschnitt aufweist.

Der Trichter kann lösbar oder unlösbar befestigt sein oder beispielsweise an den Rand der Aufnahmeöffnung angeformt sein. Ein lösbarer Trichter ist insbesondere dann sinnvoll, wenn er für die weitere Verwendung sterilisiert oder gereinigt werden soll oder wenn er aus einem Material besteht, welches gesondert recycelt werden soll. Durch die Verwendung eines gesonderten, ablösbaren Trichters verringern sich weiterhin die Abmessungen, was den Transport und die weitere Handhabung erleichtert. Die Auslauföffnung des Trichters kann dabei so groß gewählt werden, dass eine Verstopfung vermieden wird.

Vorzugsweise ist vorgesehen, dass der Filter mehrstufig ausgebildet ist und ein oder mehrere erste Filter gröbere Probenbestandteile zurückhalten und ein oder mehrere zweite Filter feinere Probenbestandteile zurückhalten, welche die ersten Filter bereits passiert haben. Insbesondere können zwei Filter, nämlich ein grober und ein feinere Filter vorgesehen sein. Die Verwendung eines mehrstufigen Filters vermeidet, dass der feinere Filter verstopft wird, bevor genügend Probe durch die Filter geflossen ist. Der Filterkuchen kann separat analysiert werden. Dabei können alle herausgefilterten Probenbestandteile wieder zusammengeführt oder der Filterkuchen jedes Filters separat analysiert werden.

Der Filter kann von verschiedenen Filtermaterialien gebildet sein. Bei einer Variante umfasst der Filter wenigstens ein Filtersieb. Das Filtersieb kann aus Metall bestehen und wiederverwertbar sein. Alternativ kann das Filtersieb aus Kunststoff bestehen und an das Adaptergehäuse angeformt sein. Dann wird das Adaptergehäuse als Ganzes entsorgt oder wiederverwertet. Auch Filterkartuschen, rückspülbare Filter und dergleichen können geeignet sein. Die Wahl des Filters hängt auch davon ab, ob der Filterkuchen einer weiteren Verwendung zugeführt werden soll oder ggf. mit dem Filtermaterial entsorgt wird. Auch die Art des Analyten kann sich auf die Wahl des Filtermaterials auswirken. Es versteht sich, dass das Filtermaterial mit Zusätzen oder anderen Materialien versehen sein kann, welche das Filtrat oder den Filterkuchen beeinflussen.

Das Adaptergehäuse mit dem Filter kann zusammen mit dem Probengefäß zu einem Labor geschickt werden. Dies ist insbesondere dann von Vorteil, wenn auch der Filterkuchen untersucht werden soll. Wenn nur das Filtrat mit dem Blut oder nur der Filterkuchen untersucht werden soll, ist es vorteilhaft, wenn das Adaptergehäuse mit dem Filter vom Probengefäß trennbar ist. Dann kann beispielsweise das Adaptergehäuse mit oder ohne Filterkuchen entsorgt oder an ein unterschiedliches Labor verschickt werden.

Eine besonders nutzerinnenfreudliche Anordnung ergibt sich, wenn ein Ständer, ein Halter oder eine stehende Umhausung vorgesehen ist, in welcher das Adaptergehäuse fest in einer Position gehalten ist, bei welcher die Probe in die Aufnahmeöffnung eingießbar ist. Anders als im Labor, wo regelmäßig eine große Vielzahl von Haltern und Ständern verfügbar ist, stehen im normalen Haushalt keine derartigen Gerätschaften zur Verfügung. Ein Adapter bei dem das Adaptergehäuse leicht aufzustellen ist, vereinfacht die Handhabung und ermöglicht so eine höhere Qualität bei der Probennahme.

Bei einer vorteilhaften Variante ist vorgesehen, dass die stehende Umhausung einen langgestreckten Körper umfasst, der auf einer planen Fläche aufstellbar ist und in welchem im unteren Bereich das Probengefäß und im oberen Bereich das Adaptergehäuse aufnehmbar ist. Dabei kann das Probengefäß und/oder das Adaptergehäuse in der Umhausung eingesteckt, angeformt, eingeklipst oder auf andere Weise gehalten werden. Wird das Probengefäß in der Umhausung gehalten, kann das Adaptergehäuse auch am Probengefäß gehalten werden oder auf dieses aufgesteckt sein. Umgekehrt kann auch das Adaptergehäuse in der stehenden Umhausung gehalten werden und das Probengefäß hängt am Adaptergehäuse.

Bei einer weiteren Ausgestaltung der Erfindung ist die Umhausung mit dem Probengefäß von den übrigen Komponenten des Adapters trennbar und das Probengefäß und/oder die Umhausung mit einem Verschluss verschließbar. Die Umhausung kann aus einem stabilen Material gefertigt sein, welches sich auch als Transportverpackung eignet. Es versteht sich, dass die Umhausung mit thermischer Isolierung, stoßfest und/oder luft- und wasserdicht ausgebildet sein kann, um einen sicheren Transport zum Labor zu gewährleisten. Die Umhausung kann lösbar und somit wiederverwertbar ausgebildet sein.

Bei einer weiteren Ausgestaltung der Erfindung sind ein oder mehrere Sensoren zum Erfassen von Proben- und/oder Umgebungseigenschaften vorgesehen. Beispiele für solche Sensoren sind Temperatursensoren, Leitfähigkeitssensoren oder Füllstandssensoren. Es können Signale mit dem Sensor erzeugbar sein, welche drahtlos an ein Endgerät mit einer Schnittstelle übertragbar sind. Beispiele für eine Drahtlosverbindung sind Bluetooth oder WiFi. Es versteht sich, dass auch jede andere Drahtlosverbindung geeignet sein kann. Der Sensor oder das Endgerät kann an das Internet oder ein anderes geeignetes Netzwerk angeschlossen sein. Die Datenverarbeitung kann an einem zentralen Server erfolgen. Es ist aber auch möglich, dass die Sensorsignale direkt mit einer geeigneten Applikation auf einem Endgerät verarbeitet werden. Dadurch wird die Übertragung von möglicherweise sensiblen Daten vermieden.

Die Probe in dem Probengefäß kann mit einer sterilen Verpackung und/oder einem Versandbeutel zum Labor geschickt werden. Sowohl auf dem Probengefäß, als auch auf einer eventuellen Umhausung und dem Versandbeutel kann eine Kennung angebracht sein. Die Kennung kann als computerlesbare Kennung, etwa als Strichcode oder QR-Code ausgebildet sein. Es ist aber auch möglich einen RFID-Chip oder jede andere Art von Identifikationsmarker zu verwenden.

Es ist möglich, den Adapter und das Probengefäß separat zur Verfügung zu stellen. Dies ist insbesondere sinnvoll, wenn mehrere Probengefäße mit dem gleichen Adapter befüllt werden sollen. Dann ist der Filter vorteilhafterweise austauschbar oder lässt sich reinigen.

Vorteilhafterweise sieht die Erfindung eine Vorrichtung zur Bereitstellung einer Blut enthaltenden Probe, enthaltend ein verschließbares Probengefäß und einen Adapter vor. Dabei kann insbesondere das Probengefäß und/oder der Adapter eine oder mehrere Substanzen oder Zusätze enthalten, die ausgewählt sind aus der Gruppe enthaltend:
(a) Puffer, chaotrope Substanzen, Rinderserum und/oder anderes Serum und/oder andere Zusatzstoffe zum Konservieren und/oder Lysieren von Endometrium-, Zervix- oder Vaginalzellen oder - zellbestandteilen
(b) Heparin, Li-Heparin, EDTA und/oder andere Koagulationshemmer;
(c) koagulationsfördernde Substanzen;
(d) Antibiotika, Natriumazid, Natriumfluorid, andere Bakteriostatine, Antimykotika und/oder andere Zusatzstoffe zum Abtöten oder Hemmen des Wachstums von Mikroorganismen;
(e) Natriumcitrat; Kaliumcitrat und/oder andere schleimverändernde Substanzen;
(f) GITC, Trizol, Kohlenhydratisolatoren und/oder andere Wirkstoffe;
sowie Zusammensetzungen, Mischungen und Derivate davon.

Die Probengefäße und/oder der Adapter können eine Farbcodierung, beispielsweise am Deckel, aufweisen, welche entsprechend der geltenden Norm als Hinweis auf die enthaltenen Substanzen oder Zusätze dienen. Die Substanzen oder Zusätze können als Beschichtung auf der Innenseite des Probengefäßes, als Gel oder als Flüssigkeit vorliegen. Es ist aber auch möglich, lösliche Feststoffe, beispielsweise in Pulver- oder Granulatform, einzusetzen.

Erfindungsgemäß erfolgt das Analysieren von Blut enthaltenden Proben nach einem Verfahren mit den Schritten:
(a) Überführen des in der Probe enthaltenen Bluts in ein Probengefäß;
(b) Verpacken und Befördern der Probe oder Teile davon zu einem Analysenlabor; und
(c) Durchführen der Analyse.

Das Verfahren ist dadurch gekennzeichnet, dass
(d) die Probe von Menstruationsausscheidungen gebildet ist;
(e) die Probe in einer Menstruationstasse oder einem anderen Behälter gesammelt wird;
(f) die gesammelte Probe zumindest teilweise direkt am Sammelort mit einem Adapter in das Probengefäß überführt wird, wobei der Adapter ein Adaptergehäuse mit einer Aufnahmeöffnung mit Abmessungen aufweist, die das manuelle Eingießen der Probe in die Aufnahmeöffnung sicher erlauben; und wenigstens ein Filter in oder an dem Adaptergehäuse vorgesehen ist, mit welchem Zellen, Gewebe, Sekret oder andere feste Bestandteile der Probe von den flüssigen Anteilen der Probe abtrennbar ist.

Zur Durchführung des Verfahrens erhält die menstruierende Person ein Kit bestehend aus:
(a) Adapter mit Adaptergehäuse und Filter;
(b) Probengefäß mit Kennung;
(c) Versandbehälter oder -tasche, optional bereits mit Rücksendeadresse versehen;
(d) optional: Ständer, Halter oder Umhausung;
(e) optional: Menstruationstasse;
(f) Gebrauchsanweisung, QR-Code mit Link oder anderen Zugang zur Gebrauchsanweisung.

Mit einem solchen Kit können erstmalig Blut enthaltende Menstruationsausscheidungen vom Laien stabil und analysenfertig aufbereitet und an ein Labor verschickt werden. Es ist kein Gang zu einem Arzt, in ein Labor oder in eine Forschungseinrichtung erforderlich.

Die Analyse kann in üblicher Weise alle bekannten Inhaltsstoffe und Eigenschaften von Blut bestimmen, beispielsweise mit Immunoassay, Massenspektrometrie, Dried Bloot Spot (DBS)-Analyse etc. sowie Biomarker wie Hormone, Spurenelemente und Vitamine.

Ausgestaltungen der Erfindung sind Gegenstand der Unteransprüche. Ein Ausführungsbeispiel ist nachstehend unter Bezugnahme auf die beigefügten Zeichnungen näher erläutert.

### Definitionen

In dieser Beschreibung und in den beigefügten Ansprüchen haben alle Begriffe eine dem Fachmann geläufige Bedeutung, welche der Fachliteratur, Normen und den einschlägigen Internetseiten und Publikationen, insbesondere lexikalischer Art, beispielsweise www.Wikipedia.de, www.wissen.de oder der Wettbewerber, forschenden Institute, Universitäten und Verbände dargelegt sind. Insbesondere haben die verwendeten Begriffe nicht die gegenteilige Bedeutung dessen, was der Fachmann den obigen Publikationen entnimmt.

### Kurze Beschreibung der Zeichnungen

- Fig.1: ist eine schematische Darstellung und illustriert den Aufbau einer Probenvorbereitungsanordnung mit Probenröhrchen, Adapter und Trichter.
- Fig.2: zeigt eine Variante von Figur 1, mit einem angeformten, konischen oberen Rand des Adapters.
- Fig.3: zeigt eine Variante von Figur 2 und 3 mit einem konischen Adapter.
- Fig.4: illustriert die Probennahme mit einer Anordnung aus Figur 1.
- Fig.5: zeigt die Umhausung der Anordnung aus Figur 1 separat.
- Fig.6: ist eine Detaildarstellung der Filteranordnung für die Anordnung aus Figur 1.
- Fig.7: illustriert, wie das Probenröhrchen und die Umhausung einer Anordnung aus Figur 1 verschlossen werden.
- Fig.8: zeigt eine alternative Ausgestaltung der in Figur 1 gezeigten Anordnung ohne Umhausung.
- Fig.9: ist eine Explosionsdarstellung der Anordnung aus Figur 8.

### Beschreibung der Ausführungsbeispiele

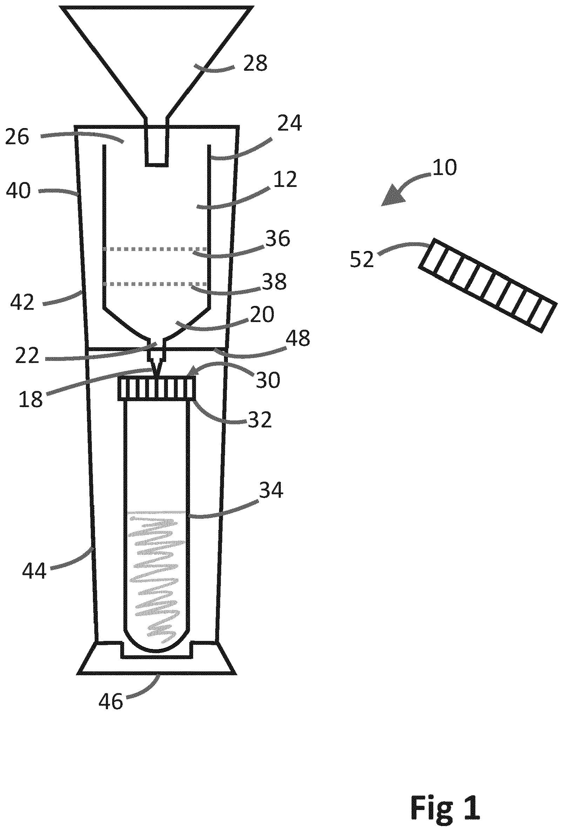

Figur 1 zeigt eine Anordnung zum Sammeln von Menstruationsausscheidungen, die allgemein mit 10 bezeichnet ist. Die Anordnung 10 ist mehrteilig ausgebildet. Ein Adapter 12 umfasst ein Adaptergehäuse 14 und eine Überführungshilfe 16 mit einer Nadel 18. Das Adaptergehäuse ist im Wesentlichen zylindrisch ausgebildet und weist ein konisches unteres Ende 20 mit einer Öffnung 22 auf. Am oberen Ende 24 des Adaptergehäuses 14 ist eine Öffnung 26 vorgesehen. Im vorliegenden Ausführungsbeispiel ist ein Trichter 28 in die Öffnung 26 eingesteckt.

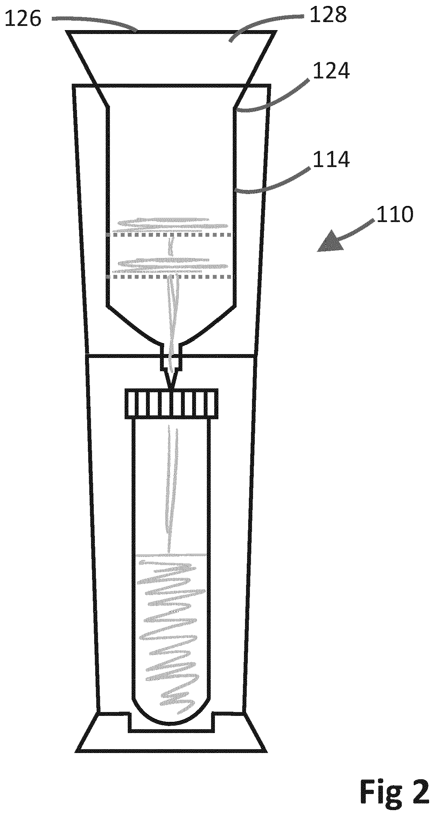

Bei einem ansonsten identischen, alternativen Ausführungsbeispiel 110 ist ein trichterförmiger Rand 128 an das obere Ende 124 des Adaptergehäuses 114 angeformt. Dies ist in Figur 2 dargestellt.

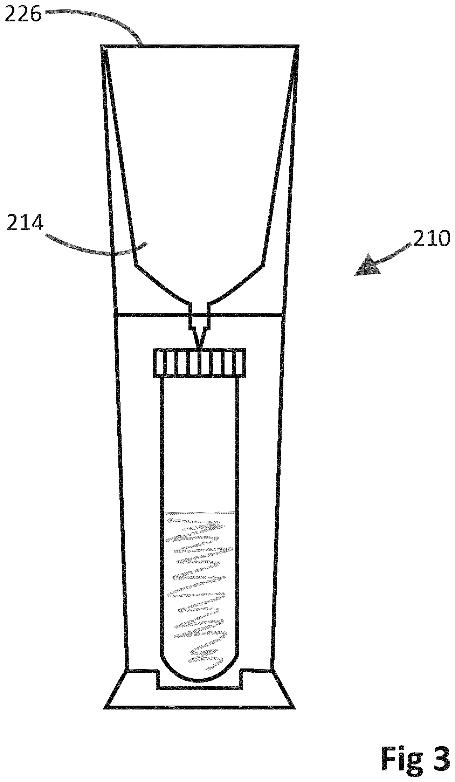

Bei einem weiteren, alternativen Ausführungsbeispiel 210 ist das Adaptergehäuse 214 nicht zylindrisch, sondern konisch aufgeweitet, so dass die obere Öffnung 226 einen vergrößerten Durchmesser hat. Dieses Ausführungsbeispiel ist in Figur 3 illustriert.

Die obere Öffnung 126 bzw. 226 oder der in die obere Öffnung eingesteckte Trichter 28 haben Abmessungen, welche das leichte Eingießen von Menstruationsausscheidungen erlauben. Im vorliegenden Ausführungsbeispiel hat die Öffnung einen Durchmesser von etwa 2,5 cm bis ca. 4 cm.

Die Menstruationsausscheidungen werden im vorliegenden Ausführungsbeispiel mit einer Menstruationstasse 29 gesammelt. Es versteht sich, dass auch andere Einrichtungen verwendet werden können, welche das Sammeln von Menstruationsausscheidungen erlauben. Dies müssen nicht zwingend in das Körperinnere eingeführt werden.

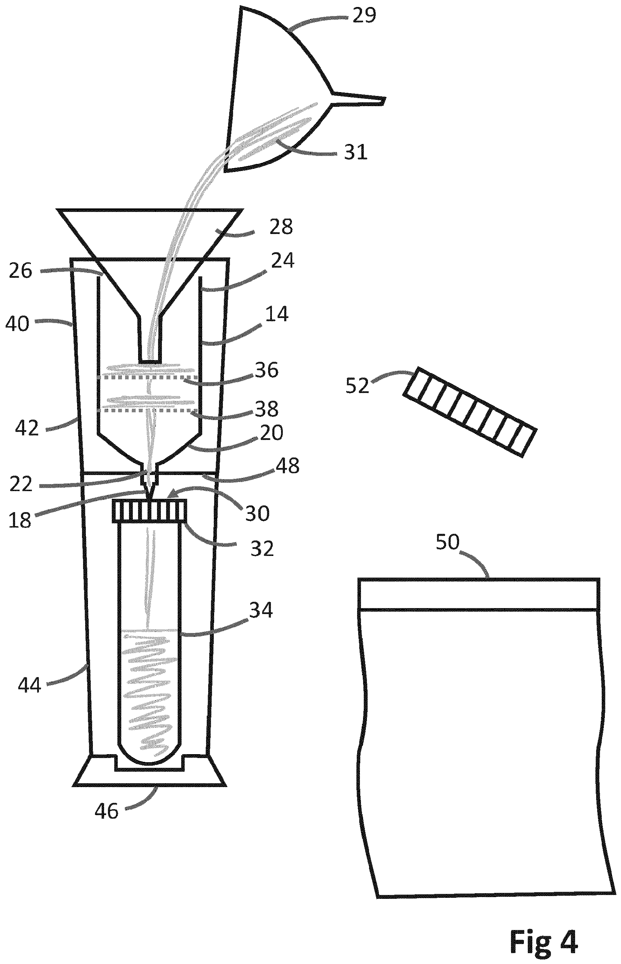

Die gesammelten Menstruationsausscheidungen bilden eine Blut enthaltende Probe 31, die aus der Menstruationstasse in den Adapter gegossen werden. Dies ist beispielhaft in Figur 4 illustriert. Die in den Menstruationsausscheidungen enthaltenden festen oder schleimigen Komponenten können mit dem Blut ohne Gefahr des Verschüttens in die die Trichteröffnung des Trichters 28 bzw. bei den anderen Ausführungsbeispielen in die vergrößerte obere Öffnung 126 bzw. 226 bzw. gegossen werden. Handelsübliche Menstruationstassen 29 bestehen aus flexiblem Silikonmaterial. Dies erlaubt ein leichtes Zusammendrücken, so dass eine Ausgießtülle gebildet werden kann, mit welcher die Menstruationsausscheidungen zielgenauer in die Öffnung bzw. den Trichter 28 befördert werden können.

Die untere Öffnung 22 des Adaptergehäuses 12 mündet in einer Hohlnadel 18. Die Hohlnadel 18 ist über ein Verbindungsstück 16 mit dem Adaptergehäuse 12 verbunden. Die Hohlnadel 18 ist zunächst steril mit einer Kappe oder einem Gummisiegel versiegelt. Die Versiegelung wird zur Benutzung entfernt.

Die Hohlnadel 18 dient zum Durchstechen einer Membran 30 in einem Deckel 32, welcher einen Probenbehälter in Form eines mit Unterdruck beaufschlagten Probenröhrchens 34 verschließt. Sobald die Hohlnadel 18 die Membran 30 durchsticht, wird die im Adaptergehäuse 12 befindliche Probe durch die Hohlnadel 18 in das Probenröhrchen 34 gesaugt.

Im Adaptergehäuse 12 sind zwei hintereinandergeschaltete Filter 36 und 38 angeordnet. Die Filter werden von zwei Sieben 36 und 38, beispielsweise aus Metall oder Kunststoff, gebildet, die separat entnommen werden können. Das stromaufwärtige Sieb 36 hat eine größere Maschenweite als das stromabwärtige Sieb 38. Dadurch wird vermieden, dass das feinere Metallsieb 38 zu schnell verstopft. Es versteht sich, dass auch Siebe aus anderen Materialien verwendet werden können.

Die Filter 36 und/oder 38 können nach der Verwendung gemeinsam mit dem Filterkuchen in ein oder mehrere Zell-Probenröhrchen überführt und zur Analyse in ein Labor geschickt werden. Die Zell-Probenröhrchen können wie das Adaptergehäuse 12 zur Probenvorbereitung beschichtet oder mit Zusätzen versehen sein. Geeignet sind Puffer mit Serum (Rind, Pferde oder andere), bakterizide Mittel, bakteriostatische Mittel, fungizide Mittel zur Unterstützung der Konservierung endometrialer/zervikaler/vaginaler Zellbestandteile oder mikrobieller Flora für die Diagnostik,Therapie oder Forschung. Es können weiterhin Wirkstoffe zum Auflösen und/oder Aufbrechen endometriale/zervikale/vaginale Zellbestandteile oder mikrobielle Flora für Diagnostik, Therapeutik oder Forschung verwendet werden. Beispiele sind chaotrope Mittel wie GITC und Trizol, Nukleinsäuren zur Säure-, Eiweiß- und/oder Kohlenhydratreinigung.

Mit Resten der Menstruationsausscheidungen, die in der Hohlnadel 18 verblieben sind, können Proben auf saugfähigem Filterpapier für die DBS-Analyse aufgebracht werden.

Bei einem alternativen Ausführungsbeispiel bestehen die Filter 36 und 38 aus dem gleichen Kunststoff, wie das Adaptergehäuse und sind auf der Innenseite des Adaptergehäuses angeformt oder fest angeklebt oder eingeklipst. Dann können sie gemeinsam mit dem Adaptergehäuse 12 nach der Verwendung entsorgt werden.

Im vorliegenden Ausführungsbeispiel enthält das Adaptergehäuse 12 Zusatzstoffe. Die Zusatzstoffe dienen zur Probenaufbereitung und werden in Abhängigkeit von der späteren Analyse oder zur weiteren Verwendung in der Diagnostik oder Therapie ausgewählt. Beispiele für solche Zusatzstoffe sind Zusammensetzungen, welche Koagulation verhindern, schleimverändernde Eigenschaften haben, Mikroorganismen abtöten oder deren Wachstum hemmen, konservieren oder endometriale, zervikale und/oder vaginale Zellkomponenten lysieren.

Beispiele für Zusatzstoffe zur Verhinderung oder Unterstützung von Koagulation sind Heparin, Li-Heparin und EDTA. Beispiele für Zusatzstoffe mit schleimverändernden Eigenschaften sind Natriumcitrat und Kaliumcitrat. Beispiele für Zusatzstoffe zum Abtöten oder Hemmen des Wachstums von Mikroorganismen sind Antibiotika, Bakteriostatine, etwa Natriumazid oder Natriumfluorid, und Antimykotika. Beispiele für Zusatzstoffe, die dazu dienen Endometrium-, Zervix- und/oder Vaginalzellen zu konservieren oder zu lysieren sind Puffer, die chaotropes Rinderserum oder anderes Serum enthalten. Solche Wirkstoffe sind beispielsweise GITC und Trizol und Kohlenhydratisolatoren. Solche Zusatzstoffe liegen im Adaptergehäuse 12 in fester, gelförmiger oder flüssiger Form vor oder sind als Beschichtung auf der Innenfläche des Adaptergehäuses 12 aufgetragen.

Optional enthält der Adapter einen Sensor zum Messen von biologischen Substanzen oder Attributen der Probe. Der Sensor kann drahtlos, beispielsweise über Bluetooth oder WiFi, mit einem Endgerät, etwa einem Smartphone oder Tablet kommunizieren. Von dort werden die Signale in verarbeiteter oder unverarbeiteter Form ggf. mit weiteren Informationen an einen entfernten Server oder eine Anwendung auf dem Endgerät zu übertragen.

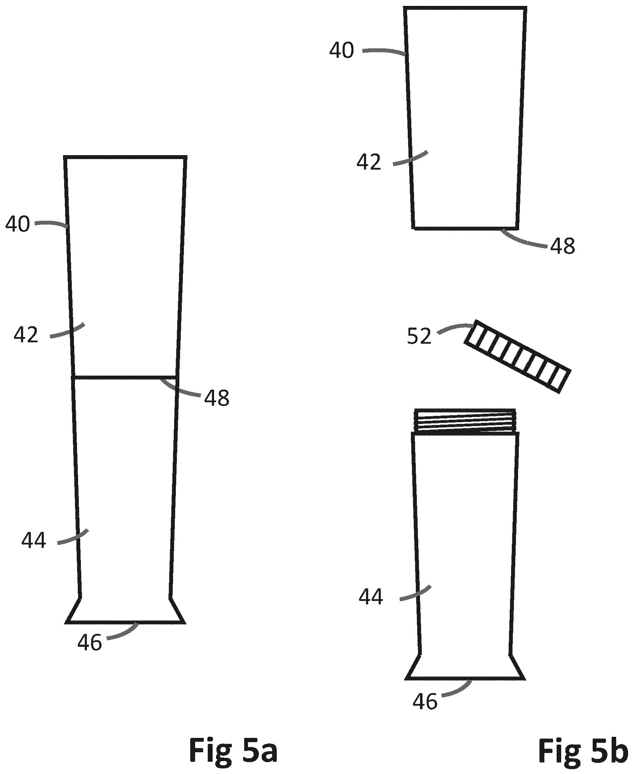

Das Adaptergehäuse 12 mit der Hohlnadel 18 bildet eine obere Komponente, die in dem oberen Teil 42 einer Umhausung 40 gehalten ist. Diese ist in Figur 5 im Detail dargestellt. Die obere Teil der Umhausung 40 ist unten offen und weist einen ebenen Rand 48 auf, so dass er mit der oberen Komponente fest auf einer planen Fläche aufstellbar ist. Der obere Teil 42 der Umhausung 40 ist mit einem unteren Teil 44 der Umhausung 40 fest verbindbar, beispielsweise verschraubbar. Der untere Teil 44 der Umhausung 40 ist mit einem Fuß 46 versehen. Mit dem Fuß 46 ist auch der untere Teil 44 mit oder oberen Teil 42 der Umhausung 40 auf einer planen Fläche aufstellbar. Die Umhausung 40 kann sowohl im zusammengesetzten Zustand, als auch getrennt als sterile Verpackung dienen oder steril verpackt sein.

Die Anordnung arbeitet wie folgt:
Die mit einer Menstruationstasse gesammelten Ausscheidungen werden in das Adaptergehäuse 12 gegossen. Mit Durchstechen der Membran 30 wird die die Ausscheidungen enthaltende Flüssigkeit mit dem Menstruationsblut durch die Filter 36 und 38 zur Hohlnadel 18 und in das Probengefäß 34 gesaugt.

Das Probenröhrchen 34 ist mit einer computerlesbaren Kennung, etwa einem Strichcode - auch als Barcode bezeichnet- , einem QR-Code, einem RFID-Chip oder einer anderen Kennung versehen. Die Probengefäße werden mit einem Deckel 52 verschlossen in einem Versandbeutel 50 an ein geeignetes Labor geschickt. Der Versandbeutel 50 ist ebenfalls mit einer computerlesbaren Kennung in Form eines Strichcodes, QR-Codes, RFID-Chip oder anderen ID versehen.

Die Daten der Analyse werden von der diagnostischen -, therapeutischen - oder Forschungseinrichtung verarbeitet und an einen Server, eine Website, ein Smartphone, ein Tablet oder eine andere Computeranwendung übertragen.

Die Anordnung verringert die Auswirkungen von Unterschieden zwischen einzelnen Proben auf den Nutzen und die Zuverlässigkeit der Analyse von Analytkonzentrationen. Auch der Einfluss des Probenvolumens und der Probenviskosität auf die Analysenergebnisse kann verringert werden. Vaginal- und Gebärmutterhalssekrete, Gewebe und Zellen des Endometriums stören nicht mehr, weil sie vor der Analyse abgetrennt wurden und für die separate Analyse zur Verfügung stehen.

Die Probennahme kann zu jeder Zeit oder an jedem Ort auf einfache und hygienische Weise erfolgen. Die gewonnenen Proben erlauben eine Vielzahl von Analysentechniken, beispielsweise Immunoassay, Massenspektrometrie, DBS-Analyse und dergleichen. Die zeitnahe Überführung der Probe in das Probenröhrchen mit Unterdruck verhindert die störende Oxidation und Kontamination der Probenbestandteile. Die Verwendung des Filterkuchens für die weitere Forschung bietet eine Alternative zu invasiven chirurgischen Eingriffen, Biopsien oder Ausschaben. Die Probenvorbereitung durch die beiden Filter verringert das Risiko für Laborgeräte bei der Analyse und insbesondere die Verstopfung von Filtern.

Die reihenweise Untersuchung von Menstruationsausscheidungen liefern Daten speziell zu Menstruierenden. Dadurch können Einblicke in den Menstruationszyklus, die Menstruation, die reproduktive und menstruelle Gesundheit, in geschlechtsspezifische Erkrankungen und die Auswirkung der Menstruation und der Phase des Menstruationszyklus auf Erkrankungen und Biomarker dieser Erkrankungen, wie beispielsweise Cholesterin oder CVD gewonnen werden. Es können ferner neue Biomarker anhand von Menstruationsblut oder anderen Bestandteilen der Menstruationsflüssigkeit identifiziert werden. Bereits bei der Probennahme können alle sichtbaren Merkmale des Menstruationsflusses, etwa Farbe, Viskosität, Dichte und Gerinnsel bewertet werden. Die Erhebung von Daten über Frauen erlauben die Untersuchung mittels Merkmalen der Menstruationsflüssigkeit zu Erkrankungen, eingenommenen Verhütungsmitteln, Medikamenteneinnahme, sexueller Aktivität und Lebensstil.

Der Adapter 12 unterstützt so die Überführung des aufbereiteten Menstruationsabflusses in standardisierte Blutentnahmeröhrchen.

Menstruationsausscheidungen enthalten eine Vielzahl biologischer Komponenten. Dazu gehören neben Vollblut, Zellen des Fortpflanzungstraktes, Endometriumgewebe sowie Vaginal- und Zervixsekrete. Während des Menstruationszyklus werden die Menstruationsausscheidungen beginnend mit dem Einsetzen der Menstruation periodisch abgeführt.

Bis vor kurzem galt Menstruationsflüssigkeit als Abfallprodukt ohne biologische Bedeutung. In den oben aufgeführten Studien konnte gezeigt werden, dass dies nicht der Fall ist. Eine Reihe von Analysen belegen, dass Menstruationsflüssigkeit eine für das Verständnis der Frauengesundheit wertvolle biologische Flüssigkeit ist.

Menstruationsblut ist weiterhin eine Quelle für Stammzellen und bildet ein potentielles, nicht-invasives Verfahren für die Stammzelltherapie. Menstruationsausscheidungen können mit einer Menstruationstasse gesammelt und daraus mononukleären Zellen gewonnen werden. Daraus gebildete mesenchymale Stammzellen (MSCs) bieten Differenzierungspotenzial.

Menstruationsausscheidungen enthalten Material und Flüssigkeit aus der späten sekretorischen Phase des Menstruationszyklus. Gewebe und Zellen stammen sowohl aus der Gebärmutterschleimhaut als auch aus dem Gebärmutterhals. Diese zusätzlichen Bestandteile im Vergleich zu Vollblut machen Menstruationsausscheidungen zu einer interessanten Flüssigkeit für die Analyse. Es erlaubt die Identifizierung neuer Biomarker für Erkrankungen des Fortpflanzungstraktes, wie Endometriose und Gebärmutterhalskrebs. Ein Beispiel ist die Zellzusammensetzung innerhalb der Menstruationsausscheidungen. Es gibt nach dem heutigen Stand der Forschung 385 Proteine, die in Menstruationsausscheidungen, aber nicht im Vaginalausfluss und im venösen Blut vorhanden sind.

Viele Eigenschaften und Bestandteile von Menstruationsblut korrelieren in hohem Grade mit systemischem Blut. Menstruationsausscheidungen können daher routinemäßig effektiv und zuverlässig für viele gängige Biomarker verwendet werden. Es kann zu Diagnose- und Gesundheitsüberwachungszwecken analysiert werden.

Bei den vorliegenden Ausführungsbeispielen werden Menstruationsausscheidungen mit einer Menstruationstasse gesammelt. Dies vermeidet Abfall und ist nachhaltiger als die Verwendung von Binden.

Die Ausführungsbeispiele zeigen eine vom Laien einsetzbare Anordnung zum Sammeln, Verarbeiten und Lagern von Menstruations- und Vaginalausscheidungen, welche Diagnostik, Therapie und Forschung ermöglichen.

Die Menstruationsflüssigkeit enthält Endometriumzellen, Schleim, Zelltrümmer, Gebärmutter-, Zervix- und Vaginalsekret sowie vaginale Mikroflora. Je nach Zeitpunkt der Probenentnahme und Individuum liegen unterschiedliche Mengen der Bestandteile vor. Es werden alle diese Bestandteile weitestgehend herausgefiltert, so dass sie die diagnostischen Analyseverfahren nicht stören.

Der flüssige Teil der Probe wird mittels des Adapters in Blutentnahmeröhrchen überführt. Solche Blutentnahmeröhrchen sind kommerziell verfügbar. Es können aber auch speziell angepasste Blutentnahmeröhrchen verwendet werden. Die Blutentnahmeröhrchen sind geeignet für die routinemäßige Verwendung im Labor, um beispielsweise Biomarkerspiegel zu analysieren. Gleichzeitig werden die Zellbestandteile im Filterkuchen separat gesammelt und können für therapeutische und Forschungszwecke eingesetzt werden. Der Adapter hilft bei der Probenentnahme zu Hause. Er kann aber auch in diagnostischen-, medizinischen- und Forschungseinrichtungen verwendet werden.

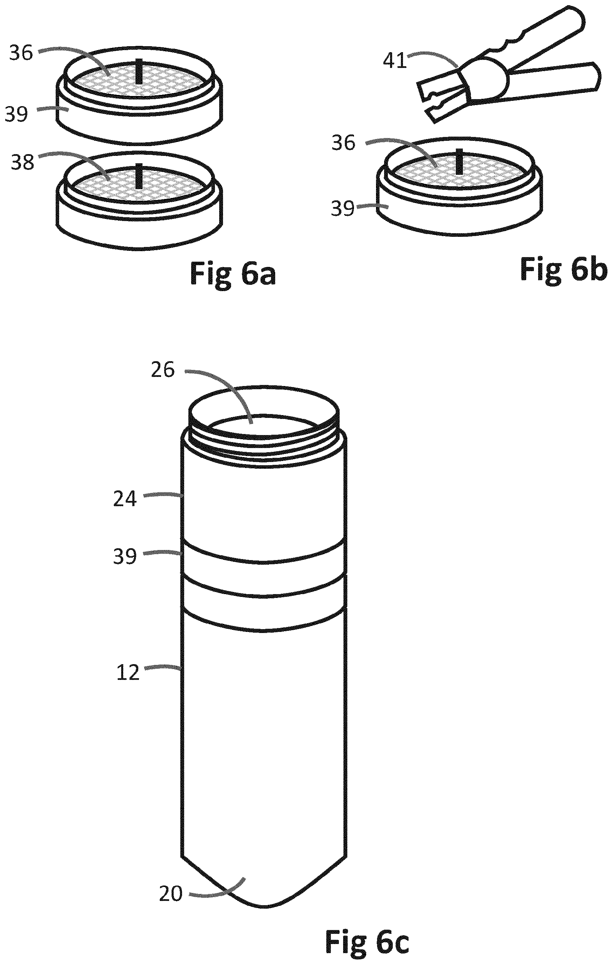

Figur 6 illustriert, wie die Filter 36 und 38 ausgetauscht oder gereinigt werden können. Jeder Filter 36 bzw. 38 ist in einem ringförmigen Gehäuse 39 beispielsweise auf einer Ringschulter auf der Innenwandung gehalten. Die ringförmigen Gehäuse 39 sind stapelbar. Dies ist in Figur 6a illustriert. Figur 6b zeigt ein separates Gehäuse 39. Die Übrigen Teile 12 und 24 können auf gleiche Weise mit dem Stapel verbunden werden. Dies ist in Figur 6c illustriert. Die Verbindung kann geklipst, geschraubt oder gesteckt werden. Es versteht sich, dass auch weitere Filter in weiteren Gehäusen aufgesteckt werden können. Zum Lösen der Verbindung kann ein Werkzeug 41 verwendet werden. Wenn dies nur mit einem eigens hierfür vorgesehenen Werkzeug möglich ist, kann verhindert werden, dass ein Laie die Verbindung löst und Probenteile kontaminiert werden. Mit einem solchen modularen Aufbau kann der Filterkuchen zur weiteren Analyse zur Verfügung gestellt werden.

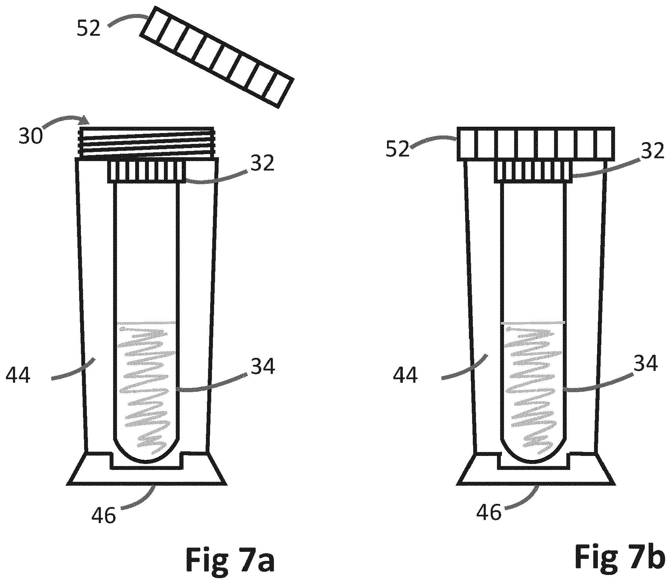

Figur 7 illustriert, wie die gefilterte Probe versandfertig verschlossen werden kann. Der untere Teil 44 der Umhausung wird vom oberen Teil gelöst und das Probennahmeröhrchen 34 mit einem Deckel 32, beispielsweise einem Schraubdeckel verschlossen. Dies ist in Figur 7a illustriert. Dann wird der untere Teil 44 der Umhausung ebenfalls mit einem Deckel 52, beispielsweise einem Schraubdeckel, verschlossen. Dies ist in Figur 7b illustriert. Zum Zentrifugieren und/oder Analysieren können die Deckel nach dem Transport im Labor leicht entfernt werden.

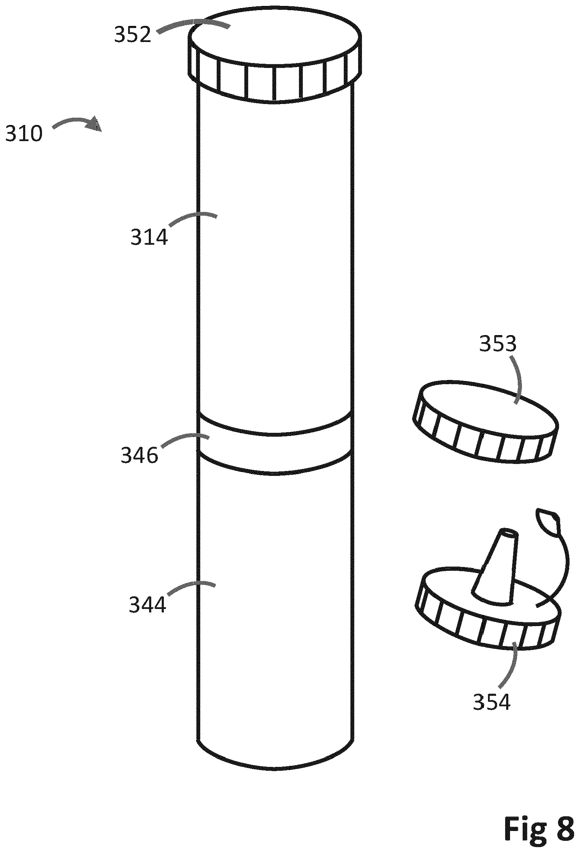

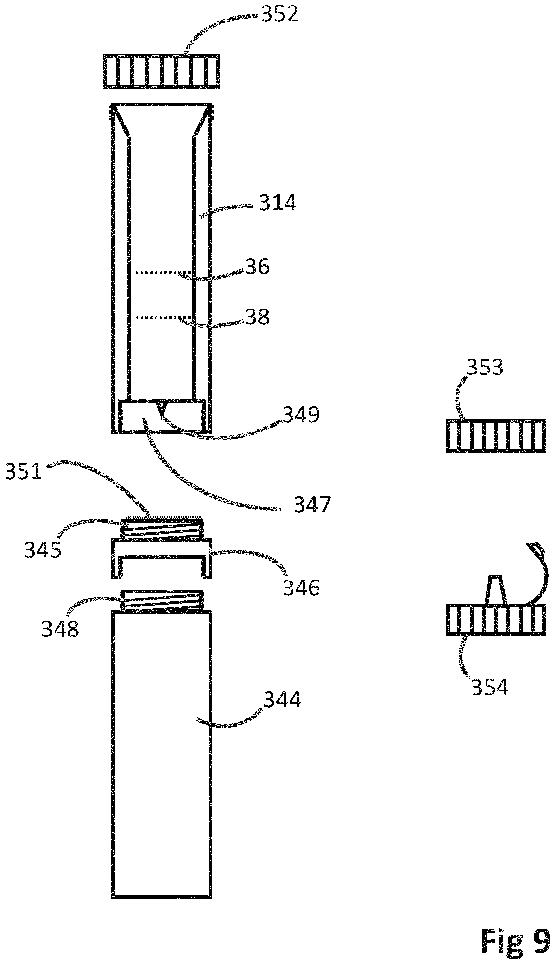

Figur 8 und Figur 9 illustrieren eine alternative Lösung zum Ausführungsbeispiel aus Figur 7. Die Anordnung 310 ist ebenfalls mehrteilig ausgebildet, hat aber eine geringere Baulänge in Längsrichtung. Ein mit einem Deckel 352 verschließbarer oberer Gehäuseteil 314 ist auf einen, ähnlich der beschriebenen Probennahmeröhrchen, mit einer Membran 351 versehenen Adapter 346 aufschraubbar. Hierfür ist ein Gewinde 345 vorgesehen, welche in Figur 9 zu erkennen ist. Das untere Gehäuseteil 344 ist bei Bereitstellung mit Adapter 346 verbunden und mit einem Vakuum versehen. Nach Nutzung kann der Adapter 346 von Gehäuseteil 344 durch das Schraubgewinde 348 gelöst werden. Der Boden 347 des oberen Gehäuseteils 314 ist nach innen zylindrisch ausgehöhlt. Dies ist in Figur 9 zu erkennen. An den Boden 347 ist eine Nadel 349 angeformt, die nach unten öffnet. Die Spitze der Nadel 349 liegt oberhalb des ringförmigen unteren Randes des oberen Gehäuseteils 314. Auf diese Weise kann der obere Gehäuseteil 314, wie auch der untere Gehäuseteil 344 auf einer ebenen Fläche aufgestellt werden. Wenn die separat bereitgestellten Gehäuseteile 314 und 344, zweiteres in Verbund mit dem Adapter 346, für die Nutzung verschraubt werden, dringt die Nadel 349 durch die Membran 351. Eine Kontamination des Inneren des Gehäuseteils 344 wird so vermieden. Das untere Gehäuseteil 344 in Verbund mit Adapter 346 kann für den Transport mit einem Schraubdeckel 353 verschlossen werden. Weiterhin kann das Gehäuseteil 344 mit einem Deckel mit Tropfaufsatz 354 verschlossen werden, um die Probe in weiteren Verarbeitungsschritten dosiert abzugeben. Bei dieser Ausführungsvariante werden weniger Bauteile mit weniger Material benötigt und die Baulänge ist geringer, so dass Handhabung, Herstellung, Transport und Lagerung vereinfacht werden. Auch bei dieser Variante können die Filter wie beschrieben in Form einer Stapelanordnung eingesetzt werden.

Die oben erläuterten Ausführungsbeispiele dienen der Illustration der in den Ansprüchen beanspruchten Erfindung. Merkmale, welche gemeinsam mit anderen Merkmalen offenbart sind, können in der Regel auch alleine oder in Kombination mit anderen Merkmalen, die im Text oder in den Zeichnungen explizit oder implizit in den Ausführungsbeispielen offenbart sind, verwendet werden. Maße und Größen sind nur beispielhaft angegeben. Dem Fachmann ergeben sich geeignete Bereiche aus seinem Fachwissen und brauchen hier daher nicht näher erläutert werden. Die Offenbarung einer konkreten Ausgestaltung eines Merkmals bedeutet nicht, dass die Erfindung auf diese konkrete Ausgestaltung beschränkt werden soll. Vielmehr kann ein solches Merkmal durch eine Vielzahl anderer, dem Fachmann geläufigen Ausgestaltungen verwirklicht werden. Die Erfindung kann daher nicht nur in Form der erläuterten Ausgestaltungen verwirklicht werden, sondern durch alle Ausgestaltungen, welche vom Schutzbereich der beigefügten Ansprüche abgedeckt sind.

Die Begriffe "oben", "unten", "rechts" und "links" beziehen sich ausschließlich auf die beigefügten Zeichnungen. Es versteht sich, dass beanspruchte Vorrichtungen auch eine andere Orientierung annehmen können. Der Begriff "enthaltend" und der Begriff "umfassend" bedeuten, dass weitere, nicht-genannte Komponenten vorgesehen sein können. Unter dem Begriff "im Wesentlichen", "vorwiegend" und "überwiegend" fallen alle Merkmale, die eine Eigenschaft oder einen Gehalt mehrheitlich, d.h. mehr als alle anderen genannten Komponenten oder Eigenschaften des Merkmals aufweisen, also bei zwei Komponenten beispielsweise mehr als 50%.

## Patentansprüche

1. Adapter (10) zum Überführen einer Blut enthaltenden Probe in ein verschließbares Probengefäß (34), **dadurch gekennzeichnet, dass**
(a) der Adapter (10) ein Adaptergehäuse (12) mit einer Aufnahmeöffnung (26) mit Abmessungen aufweist, die das manuelle Eingießen der Probe in die Aufnahmeöffnung (26) sicher erlauben; und
(b) wenigstens ein Filter (36, 38) in oder an dem Adaptergehäuse (12) vorgesehen ist, mit welchem Zellen, Gewebe, Sekret oder andere feste Bestandteile der Probe von den flüssigen Anteilen der Probe abtrennbar ist.

2. Adapter nach Anspruch 1, **dadurch gekennzeichnet, dass** der Adapter (10) eine Nadel (18) an der Unterseite des Adaptergehäuses (12) umfasst, mit welcher eine an dem Probengefäß (34) befindliche Membran (30) durchstechbar ist, so dass das gefilterte Blut durch die Nadel (18) in das Probengefäß (34) überführbar ist.

3. Adapter nach Anspruch 2, **dadurch gekennzeichnet, dass** eine zumindest teilweise lösbare Schutzhülle und/oder eine Versiegelung an der Nadel (18) vorgesehen ist.

4. Adapter nach einem der vorgehenden Ansprüche, **dadurch gekennzeichnet, dass** das Probengefäß (34) Abmessungen eines kommerziell verfügbaren Probennahmeröhrchens hat.

5. Adapter nach einem der vorgehenden Ansprüche, **dadurch gekennzeichnet, dass** die Aufnahmeöffnung von einem Trichter (28) gebildet ist, dessen Auslaufende in einem Behälter (12) mündet, der einen geringeren Querschnitt aufweist.

6. Adapter nach Anspruch 5, **dadurch gekennzeichnet, dass** der Trichter (28) lösbar oder unlösbar befestigt oder angeformt ist.

7. Adapter nach einem der vorgehenden Ansprüche, **dadurch gekennzeichnet, dass** der Filter (36, 38) mehrstufig ausgebildet ist und ein oder mehrere erste Filter (36) gröbere Probenbestandteile zurückhalten und ein oder mehrere zweite Filter (38) feinere Probenbestandteile zurückhalten, welche die ersten Filter (36) bereits passiert haben.

8. Adapter nach einem der vorgehenden Ansprüche, **dadurch gekennzeichnet, dass** der Filter (36, 38) wenigstens ein Filtersieb umfasst.

9. Adapter nach Anspruch 8, **dadurch gekennzeichnet, dass** das Filtersieb aus Metall oder Kunststoff besteht und wiederverwertbar ist.

10. Adapter nach Anspruch 8, **dadurch gekennzeichnet, dass** das Filtersieb aus Kunststoff besteht und an das Adaptergehäuse (12) angeformt ist.

11. Adapter nach einem der vorgehenden Ansprüche, **dadurch gekennzeichnet, dass** das Adaptergehäuse (12) mit dem Filter (36, 38) vom Probengefäß (34) trennbar ist.

12. Adapter nach einem der vorgehenden Ansprüche, **dadurch gekennzeichnet, dass** ein Ständer, Halter oder stehende Umhausung (40) vorgesehen ist, in welcher das Adaptergehäuse (12) fest in einer Position gehalten ist, bei welcher die Probe in die die Aufnahmeöffnung (26) eingießbar ist.

13. Adapter nach Anspruch 12, **dadurch gekennzeichnet, dass** die stehende Umhausung (40) einen langgestreckten Körper umfasst, der auf einer planen Fläche aufstellbar ist und in welchem im unteren Bereich (44) das Probengefäß (34) und im oberen Bereich (42) das Adaptergehäuse (12) aufnehmbar ist.

14. Adapter nach Anspruch 13, **dadurch gekennzeichnet, dass** die Umhausung (40) mit dem Probengefäß (34) von den übrigen Komponenten des Adapters (10) trennbar und das Probengefäß (34) und/oder die Umhausung (40) mit einem Verschluss (52) verschließbar ist.

15. Adapter nach einem der Ansprüche 1 bis 12, **dadurch gekennzeichnet, dass** der Boden des Adapters 314 nach innen gekrümmt ist und mit einer Nadel versehen ist, welche vollständig in dem durch die Krümmung gebildeten Raum aufgenommen ist, und der Adapter mit einem Probenröhrchen mit einer Membran verbindbar ist, derart, die Nadel die Membran beim Verbinden durchsticht.

16. Adapter nach einem der vorgehenden Ansprüche, **dadurch gekennzeichnet, dass** ein oder mehrere Sensoren zum Erfassen von Proben- und/oder Umgebungseigenschaften vorgesehen sind.

17. Adapter nach Anspruch 16, **dadurch gekennzeichnet, dass** Signale mit dem Sensor erzeugbar sind, welche drahtlos an ein Endgerät mit einer Schnittstelle übertragbar sind.

18. Vorrichtung zur Bereitstellung einer Blut enthaltenden Probe, enthaltend ein verschließbares Probengefäß (34) und einen Adapter (10) nach einem der vorgehenden Ansprüche, wobei das Probengefäß (34) und/oder der Adapter (10) eine oder mehrere Substanzen oder Zusätze enthält, die ausgewählt sind aus der Gruppe enthaltend:
(a) Puffer, chaotrope Substanzen, Rinderserum und/oder anderes Serum und/oder andere Zusatzstoffe zum konservieren und/oder lysieren von Endometrium-, Zervix- oder Vaginalzellen oder - zellbestandteile
(b) Heparin, Li-Heparin, EDTA und/oder andere Koagulationshemmer;
(c) koagulationsfördernde Substanzen;
(d) Antibiotika, Natriumazid, Natriumfluorid, andere Bakteriostatine, Antimykotika und/oder andere Zusatzstoffe zum Abtöten oder Hemmen des Wachstums von Mikroorganismen;
(e) Natriumcitrat; Kaliumcitrat und/oder andere schleimverändernde Substanzen;
(f) GITC, Trizol, Kohlenhydratisolatoren und/oder andere Wirkstoffe; sowie Zusammensetzungen, Mischungen und Derivate davon.

19. Verfahren zum Analysieren von Blut enthaltenden Proben mit den Schritten:
(a) Überführen des in der Probe enthaltenen Bluts in ein Probengefäß (34);
(b) Verpacken und Befördern der Probe oder Teile davon zu einem Analysenlabor; und
(c) Durchführen der Analyse;
**dadurch gekennzeichnet, dass**
(d) die Probe von Menstruationsausscheidungen gebildet ist;
(e) die Probe in einer Menstruationstasse oder einem anderen Behälter gesammelt wird; und
(f) die gesammelte Probe zumindest teilweise direkt am Sammelort mit einem Adapter (10) in das Probengefäß (34) überführt wird, wobei der Adapter (10) ein Adaptergehäuse (12) mit einer Aufnahmeöffnung (26) mit Abmessungen aufweist, die das manuelle Eingießen der Probe in die Aufnahmeöffnung (26) sicher erlauben; und wenigstens ein Filter (36, 38) in oder an dem Adaptergehäuse (12) vorgesehen ist, mit welchem Zellen, Gewebe, Sekret oder andere feste Bestandteile der Probe vom Blut abtrennbar ist.

20. Kit zur Durchführung eines Verfahrens nach Anspruch 19 enthaltend:
(a) Adapter (10) nach einem der Ansprüche 1 bis 16;
(b) Probengefäß (34) mit Kennung;
(c) Versandbehälter oder -tasche (50).

21. Kit nach Anspruch 20, zusätzlich enthaltend
(d) einen Ständer, Halter oder eine Umhausung (40); und/oder
(e) eine Menstruationstasse; und/oder
(f) eine Gebrauchsanweisung, einen QR-Code mit Link oder einen anderen Zugang zur Gebrauchsanweisung.
